(19)

**Europäisches Patentamt**

**European Patent Office**

**Office européen des brevets**

(11) **EP 4 687 153 A1**

(12) **EUROPEAN PATENT APPLICATION**

(43) Date of publication:
04.02.2026 Bulletin 2026/06

(21) Application number: 24382866.2

(22) Date of filing: 02.08.2024

(51) International Patent Classification (IPC):
*G16H 50/20* (2018.01)  *G16H 50/70* (2018.01)
*A61B 5/20* (2006.01)  *A61B 5/00* (2006.01)

(52) Cooperative Patent Classification (CPC):
**G16H 50/20; G16H 50/70;** A61B 5/208;
A61B 5/7267

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Instituto Tecnológico De Informática**
**46980 Paterna, Valencia (ES)**
• **Fundación Para la Investigación del Hospital Universitario y Politécnico La Fe de la Comunidad Valenciana**
**46026 Valencia (ES)**

(72) Inventors:
• **NAVARRO CERDÁN, José Ramón**
**E-46980 Paterna, Valencia (ES)**
• **DEL TEJO CATALA, Omar**
**E-46980 Paterna, Valencia (ES)**
• **LLOBET AZPITARTE, Rafael**
**E-46980 Paterna, Valencia (ES)**
• **PÉREZ CORTÉS, Juan Carlos**
**E-46980 Paterna, Valencia (ES)**
• **ARLANDIS GUZMÁN, Salvador**
**E-46026 Valencia (ES)**
• **COLET GUITERT, Josep Oriol**
**E-46026 Valencia (ES)**

(74) Representative: **ABG Intellectual Property Law, S.L.**
**Avenida de Burgos, 16D**
**Edificio Euromor**
**28036 Madrid (ES)**

(54) **SYSTEM FOR CLASSIFYING A UROLOGY PATIENT**

(57) The present invention comprises a training method for training a machine learning machine for classifying male urology patients into at least a first class of patients with bladder outlet obstruction (BOO) and a second class of patients with detrusor underactivity (DU). The present invention also comprises the method for classifying a urology patient into at least the first class and the second class, as well as a system configured for classifying a urology patient into at least the first class or the second class. The starting data for the classification is data representative of a uroflow measurement of a patient.

FIG. 2

**Description**

## OBJECT OF THE INVENTION

The present invention comprises a training method for training a machine learning machine for classifying male urology patients into at least a first class of patients with bladder outlet obstruction (BOO) and a second class of patients with detrusor underactivity (DU). It also comprises the method for classifying a urology patient into at least the first class and the second class, as well as a system configured for classifying a urology patient into at least the first class or the second class.

The training method for training a machine learning machine establishes the behavior of said machine in response to an input of data representative of a uroflow measurement of a patient, data acquired, for example, by means of a uroflow measurement device during urination of the patient. The training method further establishes additional parameters to be used by a method and a system adapted to subsequently carry out patient classification.

The classification method is characterized by a special technique intended for automatically extracting information from the uroflow that is not always made clearly apparent for a doctor, even if the doctor is highly capable of interpreting a uroflow curve. That is, a urologist is not capable of differentiating with certainty one pathology or another and therefore can only provide a qualitative opinion based on experience-based intuition. The classification system allows the functional cause for which a patient shows a urination deficiency to be established without the need to carry out intrusive steps, not even by means of imaging techniques.

## BACKGROUND OF THE INVENTION

One of the directions in which work is being done at the instrumental level in the acquisition of patient data is that of obtaining non-intrusive diagnostic data.

In the case of urology patients with urination difficulties, the diagnosis involves imaging tests to verify that there is no anatomical obstruction, for example due to lithiasis, tumors, stenosis, or benign prostatic growth that generate external pressure on the urinary ducts or even the growth of tissue that invades one of the ducts. In this case the section of the duct is reduced, increasing the resistance to the passage of urine.

Another cause of urination difficulty is the bladder detrusor underactivity. In this case there is a reduction in muscle contraction leading to a reduction in the pressure that is able to be generated inside the bladder to propel urine through the ducts.

In either case the result is a reduction in urinary flow.

The usual diagnostic methods involve going to the origin by checking for some of the above causes, among others.

The most common way to verify how bladder emptying occurs is for the patient to urinate using a flow meter. This causes the generation of data that can be represented in the form of a function that represents the flow and that depends on the time variable.

It is the doctor who uses his or her knowledge and experience to interpret the resulting curve, although insufficient flow may be the result of low pressure at the origin, or increased hydraulic resistance causing an obstruction.

There are artificial intelligence techniques applied to image classification that have been used in the prior art to perform classifications on curves represented in image form. These techniques depend heavily on the resolution of the image in which the curve is represented and the image with which that graphical representation is captured, limiting the capabilities to conventional classification techniques, for example by means of using neural networks.

According to the present invention, all of the above problems are solved by means of a technique that uses a machine learning machine that is trained to directly process data representative of the uroflow signal, for example, the signal produced by flow measuring means during urination of a patient.

## DESCRIPTION OF THE INVENTION

An object of a first aspect of the invention relates to the manner in which a machine learning machine which will be used for classifying patients is trained. That is, the first aspect of the invention relates to *a training method for training a machine learning machine for classifying urology patients into at least a first class of patients with bladder outlet obstruction (BOO) and a second class of patients with detrusor underactivity (DU).*

Every time reference is made in this description to the first class and to the second class, it will be according to this definition, i.e., either patients with bladder outlet obstruction or patients with detrusor underactivity. Likewise, saying that a patient belongs to the first class does not mean that a patient does not belong to the second class or that the patient suffers from another pathology causing urination difficulty.

*The method comprises the following steps for adapting the machine learning machine:*

*a) selecting a set of patients who have either been diagnosed as being in the first class or else have been diagnosed as being in the second class;*

*b) receiving data representative of a uroflow measurement during urination for each selected patient, the measurement being proportional to flow over time.*

A common process for carrying out these first two steps is the process of selecting patients who have already been diagnosed, for example, by intrusive techniques such as urodynamic studies or by means of imaging techniques. Given a patient of a certain class, the doctor must carry out a uroflow measurement, such that the uroflow data is labeled with the patient's class.

Furthermore, the following step is carried out:

*c) processing, by means of a computational system, the data received in the previous step, such that the data representative of the uroflow measurement is sampled over time, generating a vector (v) of a pre-established dimension, and wherein the set of vectors (v) of the selected set of patients results in a plurality of points in a multidimensional space, labeling each point according to the class of the patient to which it belongs.*

The uroflow data can be represented by means of a function, the measured flow, which depends on the time variable. This function is sampled with a certain sampling frequency over time such that each sample establishes a value of a vector $v$. The number of samples generated in this step of sampling defines the dimension of the vector. That is, the values of the measured flow in each sample taken over time are interpreted as the value of the coordinate in a different dimension in space. Therefore, the vector will be in a multidimensional space, the space having a dimension that is the value of the number of samples. Throughout the description it will be considered that all the vectors are generated by sampling the data representative of the uroflow measurement with the same temporal frequency. If this were not the case or even if the frequency were different, then according to a preferred example there would be an intermediate step of interpolation so that all the vectors correspond to samples in the same moment in time considering the start of the urination test to be the origin.

Each sample inherits the label identifying the class of the patient.

*d) determining, in any order, by means of a computational system:*

- *a discriminant hyperplane in the multidimensional space, such that it establishes a classification of the first class and the second class;*
- *a projection of the plurality of vectors (v) in an orthogonal projection subspace, the projection subspace in turn being orthogonal to the discriminant hyperplane, and wherein the intersection between the projection subspace and the hyperplane establishes a boundary between classes.*

Determined in these two steps is the hyperplane that verifies being a linear discriminant that divides the points in the space given by the coordinates defined by each of the vectors $v$ into two groups, a group corresponding to the first class and a second group corresponding to the second class. the orthogonal projection subspace is also defined in these two steps.

The projection is calculated after having established a scalar product, for example the usual scalar product or a weighted scalar product. Alternatives for scalar products that allow defining the direction of projection in order to apply the condition of orthogonality as well as calculate distances according to this specific scalar product will be seen below.

The orthogonal projection subspace is a subspace having a dimension smaller than the dimension of the original space given by the dimension of each vector $v$. It is considered to be of great interest the fact that the dimension of the projection subspace is much smaller than the original dimension since the coordinates of the samples projected in the projection subspace allows identifying a few variables that define coordinates which represent values that are significant in the subsequent classification into the first class or into the second class.

Likewise, by also requiring the projection subspace to be orthogonal, it results in the fact that these variables that are relevant in the classification of the projected samples into the first class and into the second class tend not to be correlated. That is, each of the variables has relevant information on its own and independently of the rest of the variables in the subsequent classification of the projected sample.

Likewise, the projection is established in a certain direction, i.e., the direction which verifies the condition of orthogonality with the orthogonal projection subspace.

Lastly, the intersection of the hyperplane with the orthogonal projection subspace establishes a boundary which is what divides the projected samples of either class on either side.

Furthermore, the following steps are carried out:

*e) determining those samples formed by vectors (v) projected in the projection subspace located at a distance from the boundary that is less than a pre-established threshold value, and removing said samples from the set of samples;*

*f) training the machine learning machine such that, for each sample of the orthogonal projection subspace, the*

*classification it provides is the class with which it is labeled, wherein the samples are the resultants in the previous step.*

There are specific cases in which the projected samples are very close to the boundary. These cases may correspond to individuals belonging to both classes, or they may be degenerate cases. These projected samples which are close to the boundary are removed when their distance is less than a pre-established distance.

According to an embodiment applicable to the described method, *the set of patients selected in step a) do not belong to both classes at the same time.*

According to this embodiment, in addition to eliminating projected samples falling close to the boundary, those patients that would be labeled in both classes at the same time and that should theoretically fall on the boundary are also removed in the selection process.

According to a specific training example, instead of removing individuals belonging to both classes, this subset of patients is used in the training phase given that they provide information about these situations where the patients suffers from both conditions at the same time. That is, it is understood that samples which correspond to patients in whom the classification is unclear fall on the boundary, and therefore they are removed, and in this case, the sample is included initially, even though the projected sample is subsequently removed because it allows more clearly defining the discriminant hyperplane.

According to an embodiment applicable to any of the examples described above, during training of the machine learning machine, vectors *v* related to patients that correspond to samples of the orthogonal projection subspace and verify that the distance to the boundary is greater than a pre-established value are discarded, as well as the sample.

Similarly to how projected samples falling very close to the boundary are removed, samples falling too far away from the boundary, according to an embodiment, are also removed, improving the training of the machine learning machine.

A second aspect of the invention relates to a *method for classifying a urology patient into at least the first class or the second class, the method using a computational system adapted to run a machine learning machine trained according to any of the examples described above, the computational system storing at least the following parameters determined in the training:*

- *parameters defining the multidimensional space,*
- *the orthogonal projection subspace, and*
- *the discriminant hyperplane and/or the boundary separating the projection space.*

That is, the training method defines the parameters that will be used in the phase of using the machine learning machine and particularly in all the steps which are common, such as the dimension of the multidimensional space where vectors built from the information representative of a uroflow measurement of a patient are expressed, the orthogonal projection subspace, the discriminant hyperplane, the boundary of projection, and others which establish the manner in which each of the projected samples have been projected in the orthogonal projection subspace.

In this case, the invention will only work with the data of a patient, i.e., the patient to be classified, *wherein the method comprises carrying out by means of the computational system, the following steps:*

*1. receiving data representative of a uroflow measurement during urination of a patient the measurement being proportional to flow over time,*

*2. processing the data from the previous step, such that the data is sampled over time, generating a vector (v) of the dimension of the multidimensional space.*

As indicated above, in conditions of use of the machine learning machine, the data representative of a uroflow measurement of the patient to be classified is sampled in the conditions in which it was carried out during training phase, that is, particularly generating the same number of samples, with the samples being measurements over time that are taken on the flow during urination of the patient. In practice and as a preferred example, the time intervals and sampling frequency for the function defining the uroflow over time used in training and in conditions of use of the machine once trained are the same.

The method according to this second aspect of the invention also comprises the following steps:

*3. projecting the vector (v) of the multidimensional space to which the vector (v) belongs in the orthogonal projection subspace, giving rise to a projected sample;*

*4. classifying the patient by means of the machine learning machine, using as input datum the sample projected on the orthogonal projection subspace depending on the side of the boundary established by the discriminant hyperplane where said projected sample is located;*

*5. providing as output the class to which the datum representative of the uroflow measurement belongs.*

Now, the vector *v* is projected in the orthogonal projection subspace where the discriminant hyperplane, as well as the boundary, are already defined. This allows identifying which side of the boundary the projected sample is on in order to classify the patient from whom the data representative of the uroflow measurement has originated.

That is, depending on the side of the boundary on which the projected sample is located, it is possible to classify the patient into the first class or into the second class, concluding the origin of the patient's pathology based solely on uroflow data without needing to use invasive or imaging tests.

Another object of this invention relates to a system comprising a computational system adapted to carry out the classification method described. That is, the third aspect of the invention relates to a system for classifying a urology patient into at least the first class or the second class.

The system according to the third inventive aspect comprises:

- *a computational system adapted to run a machine learning machine trained according to any of the examples described for the first inventive aspect and storing at least the following parameters determined in the training:*

  *parameters defining the multidimensional space,*
  *the orthogonal projection subspace, and*
  *the discriminant hyperplane and/or the boundary separating the orthogonal projection subspace,*

- *the computational system being further adapted to carry out the following steps:*

  *i. receiving data representative of a uroflow measurement during urination of a patient the measurement being proportional to flow over time,*
  *ii. processing the data from the previous step, such that the data is sampled over time, generating a vector (v) of the dimension of the multidimensional space;*
  *iii. projecting the vector (v) of the multidimensional space to which the vector (v) belongs in the orthogonal projection subspace, giving rise to a projected sample;*
  *iv. classifying the patient by means of the machine learning machine, using as input datum the sample projected on the orthogonal projection subspace depending on the side of the boundary established by the discriminant hyperplane where said projected sample is located;*
  *v. providing as output the class to which the datum representative of the uroflow measurement belongs.*

This system comprises a computational system which allows performing the steps of the classification method for classifying the patient based on the uroflow data.

According to a specific example applicable both to the method according to the second inventive aspect and to the system according to the third inventive aspect, the method and system *further comprise measuring equipment adapted to carry out a uroflow measurement during urination of the patient, the measuring equipment being adapted to generate data representative of a signal proportional to flow over time.*

According to an embodiment, this measuring equipment can be an integral part of the system according to the third inventive aspect. According to another embodiment, the data representative of the uroflow of one or more patients is stored after having been measured. This measurement generates a signal which represents the value of the urination flow of the patient over time. According to an example, this information is processed directly to be classified according to any of the examples of the second or the third inventive aspect. According to another example, this information representative of the uroflow of one or more patients is stored in a database at a time during which the extraction of more information was not possible, such as that allowed by any of the embodiments of that invention. This stored data which is what is sent to a system according to the third aspect of the invention, or it is processed by means of a method according to the second aspect of the invention for finally classifying each of the patients for whom there is information about their uroflow.

According to a specific example applicable both to the method according to the second inventive aspect described up until now and to the system according to the third inventive aspect also described up until now, the data representative of the signal measured by the uroflow measuring equipment is normalized by scaling the signal as a function of time to a pre-established period of time (T).

The normalization process establishes a common period and furthermore causes the results to be unchanging with respect to uroflow functions with different durations. The normalization process causes both the training method and the method and system for using the machine trained by the first inventive aspect to only take into consideration the specific form of the function regardless of its extension over time.

According to a specific embodiment of both the method according to any of the examples of the second inventive aspect and of the system according to any of the examples of the third inventive aspect, *the scaling is carried out by means*

*of the computational system based on the sampled signal according to the following steps:*

- *carrying out interpolation of the sampled signal by providing an interpolating function in the period of time in which the measurement is non-zero, preferably by means of piecewise polynomial interpolation or spline techniques,*
- *scaling the interpolating function from the period of measurement to the period pre-established as normalized (T);*
- *resampling the interpolating function scaled in the previous step.*

This example establishes the normalization process by generating an interpolated function by means of splines, a piecewise polynomial function with continuity both in the function and at least in the first and second derivative. This function is "compressible" in the time variable. That is, a correspondence can be established between any time interval and the normalized time in which the normalized function, for each discrete value which corresponds to a coordinate of the multidimensional space, takes the value of the function interpolated by means of splines after having scaled the function to the time interval considered as normalized.

This normalization technique, in addition to allowing the use of a single normalized period of time, uses an interpolation technique which imposes conditions of continuity in the function and at least in its first two derivatives; that is, in the slope of the function and in the curvature of the function, allowing at the same time the most relevant information of the original function to be maintained.

According to an embodiment applicable to any of the embodiments of the second inventive aspect and applicable to any of the embodiments of the third inventive aspect, *the vector v is dimensionally expanded by means of the computational system by adding the result of sampling one or more of the following functions generated from the measured signal interpreted as a function of time:*

- *the time derivative function of order n of the measured signal, with n being a natural number;*
- *the Fourier transform of the measured signal;*
- *one or more wavelet transforms of the measured signal.*

In principle, the uroflow function includes all the relevant information. However, the application of the transform or the calculation of functions such as the first derivative or any of the higher order derivatives show various, apparently hidden aspects. According to this embodiment, these functions generated from the original function are concatenated to the original function, giving rise to a new function which is representative of the uroflow of the patient but which has been enriched piecewise, with each piece including any of the identified functions. The resultant function is longer and the dimension of the multidimensional space is also bigger. Subsequently, the projection process again reduces the dimension of the original multidimensional space, which is now bigger, but this projection is established on an enriched function which means that aspects not seen in the original function are now revealed as relevant by means of the inclusion of the enriched information.

The operation of expanding the original vector must be done both in the training phase according to the first aspect of the embodiment and in the phase of use according to either second aspect of the embodiment or the third aspect of the embodiment.

According to an embodiment applicable to any of the embodiments of the second inventive aspect and applicable to any of the embodiments of the third inventive aspect, *the vectors v projected on the orthogonal projection subspace and which verify that the distance to the boundary is less than a pre-established threshold value are considered unclassifiable.*

According to this embodiment, it is considered that there is not enough certainty in the classification process when, after performing the projection process, the projected sample is located very close to the boundary. This allows increasing the validity of the classified results given that if there is not enough certainty, the classification is not considered to be valid.

According to an embodiment applicable to any of the embodiments of the second inventive aspect and applicable to any of the embodiments of the third inventive aspect, *during the classification of a patient vectors (v) projected on the orthogonal projection subspace and which verify that the distance to the boundary is greater than a pre-established threshold value are considered unclassifiable.*

This embodiment requires that the patients to be classified must be in conditions similar to the conditions of the patients used during training. This check to see if they belong to the training group is verified in terms of distance, when the distance to the boundary is greater than a certain value it is considered that the patient does not belong to the training group and is discarded, so the classification of the patient cannot be carried out.

According to an embodiment applicable to any of the embodiments of the second inventive aspect and applicable to any of the embodiments of the third inventive aspect, *the measurement of the distance d between a point x in the multidimensional space and a point in the discriminant hyperplane $x_d$ is determined by means of:*

$$d = (x - x_d)^T \beta \Omega (x - x_d)$$

where $\beta$ is a diagonal matrix with the dimension n of the multidimensional space and the elements $\beta_{ii}$ of the diagonal comprising weights assigned to each dimension; and $\Omega$ is a symmetrical square matrix nxn where each element $\Omega_{ij}$ is the positive or negative quantitative relationship between dimensions i and j.

Particularly, point x in the multidimensional space and a point in space is the measurement between the point established by a vector *v* and the discriminant hyperplane to be determined. That is, the scalar product weighted by $\beta\Omega$ determines a way to measure distances and angles that conditions the calculation of said hyperplane.

Furthermore, once the hyperplane is determined, a point corresponding to a sample projected on the orthogonal projection subspace has a measurement determined this specific way and it likewise modifies the manner in which it is determined when it is close or not close to the boundary.

It has been found that by using this distance criterion, coordinates which are apparently close are not relevant in the measurement of the distance to the boundary, and coordinates which are apparently far away from the boundary are found to determine a short distance to the boundary.

It has been verified that the use of this specific measurement of distances allows excluding projected samples which considerably improve the classification since in the projected orthogonal space the criterion takes into consideration values of coordinates that really affect the classification.

According to an embodiment applicable to any of the embodiments of the second inventive aspect and applicable to any of the embodiments of the third inventive aspect, *$\beta$ is the identity matrix I and $\Omega = \Sigma^{-1}$, with $\Sigma$ being the covariance matrix.*

This embodiment establishes a measurement having the advantages described above, but furthermore, it gives rise to a smaller number of calculations that mean that the determination thereof requires a considerably lower computational cost.

According to an embodiment applicable to any of the embodiments of the second inventive aspect and applicable to any of the embodiments of the third inventive aspect, *$x_d$ is estimated by $\mu$, the vector of dimension n representing the mean of all the individuals for each variable*.

This is a specific way to allow a suitable calculation for $x_d$.

According to an embodiment applicable to any of the embodiments of the second inventive aspect and applicable to any of the embodiments of the third inventive aspect, *the projection on the projection space is carried out by means of a PLS algorithm (i.e., partial least square, a regression method)*.

According to an embodiment applicable to any of the embodiments of the second inventive aspect and applicable to any of the embodiments of the third inventive aspect, *the calculation of the likelihood of belonging to either class during the classification is carried out by means of one of the following algorithms:*

- *PLS,*
- *logistic regression,*
- *SVM,*
- *random forest.*

A fourth inventive aspect relates to a computer program comprising instructions which, when the program is run by the computer, causes the computer to carry out steps b) to f) according to any of the machine learning machine training examples.

A fifth inventive aspect relates to a computer problem comprising instructions which, when the program is run by the computer, causes the computer to carry out steps 1 to 5 according to any of the embodiments of the second inventive aspect.

## DESCRIPTION OF THE DRAWINGS

These and other features and advantages of the invention will become more apparent based on the following detailed description of a preferred embodiment, given only by way of illustrative and non-limiting example, in reference to the attached figures.

Figures 1A-1D       These figures show an example of normalization of the uroflow function using the interpolation technique for generating functions with a different number of samples, where it can be seen how the specific characteristics of the function are maintained.

Figure 2              This figure shows the plurality of classified points for a machine trained by filtering samples in the training phase.

Figures 3A-3D      These graphs show results of four different algorithms for the projection in the orthogonal projection subspace during training, showing specificity versus sensitivity when filtering has not been applied.

Figures 4A-4D      These graphs show results of four different algorithms for the projection in the orthogonal projection subspace during training showing specificity versus sensitivity when filtering has been applied.

Figures 5A and 5D   These graphs show results of four different algorithms during the validation phase after applying the training method. The graphs show specificity versus sensitivity.

Figures 6A and 6D   These graphs show results of four different algorithms during the validation phase after applying the training method. The graphs show specificity versus sensitivity.

Figures 7A and 7D   These graphs show results of four different algorithms during the validation phase after applying the training method. The graphs show specificity versus sensitivity.

## DETAILED DESCRIPTION OF THE INVENTION

According to the first inventive aspect, the present invention relates to a training method for training a machine learning machine for classifying urology patients, specifically male patients, into at least a first class of patients with bladder outlet obstruction (BOO) and a second class of patients with detrusor underactivity (DU). It also relates to the classification method for classifying patients, also specifically male patients, by using the trained machine, as well as to a system which allows automated classification also using the trained machine.

As identified above, two main non-exclusive causes produce the problem of non-emptying bladder in men, these causes being 1) bladder outlet obstruction, and 2) detrusor underactivity. Both cases result in decreased flow rate and potentially incomplete emptying.

Bladder outlet obstruction can be relieved by means of surgery to remove the obstruction. However, this solution is not useful in detrusor underactivity. Distinguishing between both cases is crucial for offering the best treatment to the patient.

In the state of the art, there are two possible dynamic tests for urination which try to place the patient in the best group. The first test, which studies pressure and flow, is an invasive test for which bladder and rectal catheters are introduced into the patient to take different measurements, which help diagnose the patient by knowing precise measurements.

The second test, uroflow, is an easy, non-invasive test that only measures the urine stream using an external flowmeter. This flowmeter provides a time curve from measurement samples acquired every 0.1 seconds containing information about the flow rate.

The objective of the method described here is to process uroflow signals and obtain a specifically designed machine learning model which subsequently allows making a definitive decision about the group of patients to which it belongs without the need for intrusive tests on the patient.

### Material and methods

Individuals with poor bladder emptying due to underactive and obstructive emptying show different urodynamic alterations. However, the uroflows corresponding to a poor bladder emptying due to underactive emptying and obstruction are very similar given that the differences are subtle and can only be intuited by the expert urologist and without 100% reliability. Uroflow is a non-invasive technique widely used by urologists to evaluate the urination volume and urination flow speed, and it is accompanied by the measurement of post-urination residual. To that end, a flowmeter that provides information about the urinary stream dynamics is normally used. Since the data from this device physically corresponds with a time signal, while this signal could be enough, according to an optimized example different mathematical transforms related to signal processing will be used to obtain several explanatory variables which feed a classifying model for classifying the individual between both groups.

According to an embodiment, initially an individual can belong to the underactive and obstructive groups at the same time.

The method is based on a multidimensional technique where individuals who are close to the boundary are filtered out during the training process, that is, those individuals that are in a space of confusion that divides individuals between both cases are removed. Lastly, a classifying model, which will evaluate whether a specific patient is in one class or the other, that is, if he suffers from BOO or DU, will be obtained.

Removing an individual during the described method, particularly those individuals who meet a condition of distance to the boundary, must be interpreted to mean that the data corresponding to that individual is not used in subsequent steps, as his data is discarded.

*Set of data*

On one hand, a full set of 123 labeled patients has been obtained, s1, the label indicating to which class the patient belongs using the information from one or more invasive tests. The set of data acquired, s1, is made up of two groups of 61 underactive patients and 62 obstructive patients. The original data is data representative of a signal of the non-invasive uroflow test with a maximum duration of 2 minutes. On the other hand, for the example described a set of 27 distinct labeled patients, s2, 10 underactive patients, and 17 obstructive patients, has also been obtained to validate the results. Distinct labeled patients are understood to be those patients that do not suffer from both conditions at the same time, that is, they do not belong to both groups at the same time.

*Processing of the data representative of the signal*

According to this optimized example, the data representative of the original signal has been previously preprocessed by normalizing the temporal length and, subsequently, extracting explanatory characteristics of interest for the final classifier according to the technique that has been described for dimensional reduction by projection in an orthogonal projection space.

*Normalization of the size of the signal*

There are several ways to normalize a signal to a pre-established period. A very simple technique will only add zero values at the beginning or end of the signal until the pre-established period is completed. The most suitable are those that deform the signal, stretching or contracting the signal in the independent variable, adapting it to the period in an "elastic" way but by means of a transform that does not lose signal information.

In this embodiment, the application of a transform from the original signal to a set of data representative of the signal but defined in the pre-established period, with a predetermined number of sampling points and using a univariate cubic spline transform with respect to the number of input points, has been chosen. That is, the original signal is interpolated by means of splines and it is this continuous curve defined by means of piecewise polynomial functions that is transformed to the new domain. Once transformed, the piecewise polynomial function is sampled with the number of pre-established sampling points.

With this transform, subsequent operations will always operate on a space with the same pre-established dimension and without losing information with respect to the original signal, even if it does not have the same number of samples.

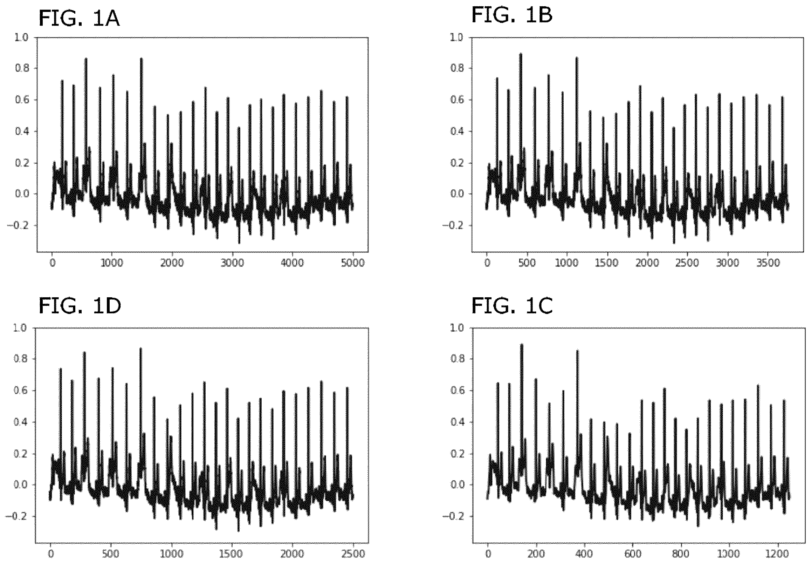

Figures 1A-1D show the result of this type of transform from the period of time corresponding to the signal capture time to a certain period of time but with a different number of samples, as indicated on the x-axis although the shape of the signal remains unchanged. Particularly, Figures 1A, 1B, 1C, and 1D show one and the same signal with 5000, 3750, 2500, and 1250 points, respectively, by using an interpolating approximation by means of univariate splines.

*Generation of enriched characteristic vectors*

According to this embodiment, the normalized original signal is enriched using various wavelet transforms although it is possible to apply additional transforms such as the first, second, third derivative, etc. Each transform gives rise to a piecewise function which is added at the end of the preceding transform. That is, the final result is a vector which essentially contains the values of the original signal after normalization, and additional components corresponding to subsequent transforms that will be concatenated at the end of the vector successively. For example, if the dimension of the vector normalized with the original signal has a dimension n1, then the addition after it of a first discrete transform by means of wavelets and the three first derivatives will result in a vector $v$ of dimension $N$ with $N = 5 * n1.$

According to another embodiment, given that the transforms by means of discrete wavelets divide the original signal into low and high frequencies and different discretization techniques by means of discrete wavelets provide values that are also different, a plurality of transforms wavelets, particularly those known as rbio3.1, bior1.1, coif1, haar, and sum4, have been chosen.

For each transform the three first derivatives (first, second, and third) have initially been calculated on the normalized original signal both in the low-frequency part with the main information of the signal and in the high-frequency part where detailed aspects of the signal and also the noise are found.

### Training phase

In this embodiment, PLS projection, which automatically determines a projection space having a smaller dimension, has been used both in the projection carried out during training and in the projection used in the method when operatively classifying patients.

However, it is possible to pre-establish a projection subspace having a smaller dimension and carry out a subsequent orthogonalization phase, for example by means of the Gram-Smidt method, such that the projection of the vector $v$ must be such that $v$ is orthogonal to any of the vectors forming base of the selected vector projection subspace.

After having established the vector projection subspace, particularly the orthogonal projection subspace, it is possible to define a set of base vectors such that any vector in the vector subspace is expressible in the new base. A vector is understood to be "expressible" in a base when it can be expressed as a linear combination of the base vectors. This same base of the vector subspace can be completed with linearly independent vectors until forming a second base of the original or complete vector space.

A particular case of base change consists of establishing a change in the axes where the first vectors of the base are those corresponding to the coordinates which provide more information, and the rest are associated with a smaller amount of information, for example because they represent noise.

According to this construction, a point belonging to the projection subspace will have a set of coordinates in the second base such that the coordinates associated with the vectors used to complete the base of the vector subspace will be zero. Therefore, it is possible to establish a restriction operation which establishes a correspondence between a vector that belongs to the orthogonal projection subspace and a vector of smaller dimension that only has the coordinates in the second base which are not always zero. This is a specific form of dimensional reduction.

The method is compatible with the specific case in which a compete base of the vector space is maintained and where the first coordinates provide more information than the last coordinates associated, for example, with noise.

It should be noted that the vector resulting from the restriction operation has the dimension of the orthogonal projection vector subspace. It is possible to select the dimension of the vector subspace as that dimension in which the number of vectors corresponds to those in which the information is relevant and the discarded vectors correspond to those in which, although the resulting coordinates are not zero, are of a value below a threshold value or correspond to noise. Also, the new base of the vector subspace in this embodiment is chosen to be orthogonal.

Given that this restricted space results from applying a restriction operation has a bijective correspondence with the orthogonal projection vector subspace, reference will be made indistinctly to either the orthogonal projection subspace or the restricted space. In the end, the result is a number of non-zero coordinates or coordinates which are considered relative to relevant values and with information which, in most cases, is smaller than the dimension of the original space.

In this way and according to a preferred example, the new restricted space only has a few dimensions where the values are not multicorrelated.

The vector $v$ originating from the measurement, whether normalized or not, has high dimensionality and its components are multicorrelated. The next step is to select an orthogonal projection subspace with a smaller dimension, preferably with a dimension considerably smaller than the original subspace where the vector $v$ is projected verifying that it is an orthogonal projection.

Going back to the training phase, in this example a patient could belong to both classes to a certain extent. In this way, the label assigned to that patient would belong to the most likely class.

Conventional classifiers are based on the assumption that individuals always belong exclusively to one class, so, although the classifier is trained in conditions of noise, what happens is that a classifier trained in these conditions can be confused trying to find discriminant functions that do not exist. On the other hand, the outlier individuals used in the training phase can change the discriminant functions and reduce the generalization of the resulting models.

In this optimized embodiment, to solve this problem, a review of the individuals used during training is carried out such that only those belonging to one of the classes and, as an additional criterion, individuals that are not very far away from one another, are selected. This second criterion is another way to optimize the results of this embodiment.

To ensure the first criterion, it is enough to consider those individuals which, after being projected on the orthogonal projection space, show a distance to discriminant hyperplane that is greater than a certain pre-established distance.

While it is possible to use the Euclidean distance, in this embodiment it has been seen that the behavior of the classifier improves, becoming more sensitive to the class to which the individual belongs by using a modified measurement where the measurement of the distance $d$ between a point $x$ in the multidimensional space and a point in the discriminant hyperplane $x_d$ is determined by means of:

$$d = (x - x_d)^T \beta \Omega (x - x_d)$$

where $\beta$ is a diagonal matrix with the dimension $n$ of the multidimensional space and the elements $\beta_{ii}$ of the diagonal comprising weights assigned to each dimension; and $\Omega$ a symmetrical square matrix $nxn$ where each element $\Omega_{ij}$ is the positive or negative quantitative relationship between dimensions $i$ and $j$.

It has been seen to be very effective and computationally simpler when $\beta$ is the identity matrix $I$ and $\Omega = \Sigma^{-1}$, with $\Sigma$ being the covariance matrix and where $x_d$ is estimated by $\mu$, the vector of dimension $n$ representing the mean of all the individuals for each variable.

Particularly this measurement is the one that has been used when applying a projection by means of a partial least square or PLS regression projection. In this case x are the first components resulting from applying the PLS method, $x_d$ are the PLS mean components, and $\Sigma^{-1}$ is the covariance matrix also of the first PLS components.

That is, the orthogonal projection space is also determined by selecting the latent or restricted space formed by the restricted latent variables determined by means of the linear regression phase in the partial least square regression method.

In this embodiment, once the distance is defined by using the covariance matrix, two threshold values are determined, with these being:

$\tau_1 = C1 + f_i(C3 - C1)$ and $\tau_2 = C3 + f_d(C3 - C1)$ where $C1$ and $C3$ are the first and third quantile, respectively, and $f_i$ and $f_d$ are constants related respectively to how close and how far away the points are with respect to boundary.

These parameters are estimated during the training phase to improve the final model which allows filtering by removing those individuals who are both far away from and close to the boundary and only considering during training those located between both parameters, that is, where $\tau_2 > d > \tau_1$.

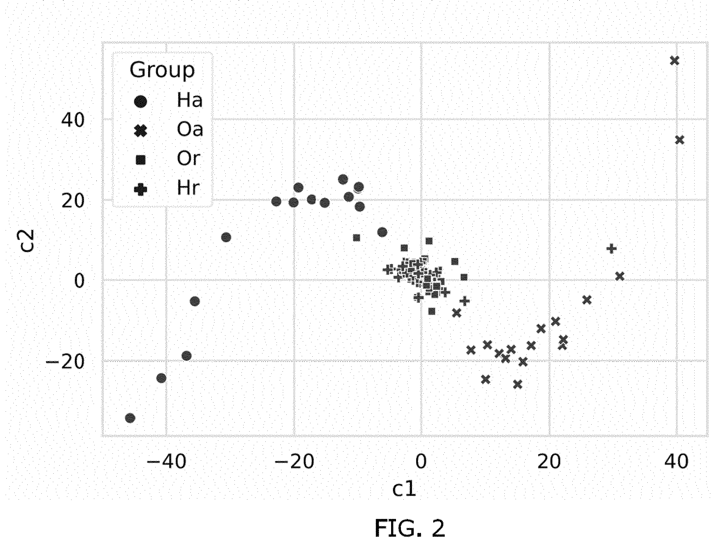

Figure 2 shows the different points classified when the learning machine is already trained, where for the data on the already identified samples, the result is $f_i = 0.75$ and $f_d = 4.0$, with these points being classified into four groups:

- To the left and with the label "Ha", these are the points classified as belonging to the first class and accepted in the filtering phase,
- To the right and with the label "Oa", these are the points classified as belonging to the second class and accepted in the filtering phase,
- In the central area and with the label "Hr" and close clustered, these are the points belonging to the first class but rejected in the filtering phase, and
- In the central area and with the label "Or" and with a certain degree of dispersion, these are the points belonging to the second class but rejected in the filtering phase.

Improvement has been observed by using various projection algorithms, with and without filtering during training phase. The result for the following four algorithms (PLSR or partial least square regression, LR or logistic regression, RF or random forest, and SVM or support vector machine) are shown in the following table where the values of the table show the of AUC (area under ROC curve) value, that is, the value of the area under ROC curve (receiver operating characteristic curve). The AUC varies in value from 0 to 1. A model with predictions that are 100% incorrect has an AUC of 0.0; a model with predictions that are 100% correct has an AUC of 1.0. Likewise, a value of 0.5 in a two-class problem with the same likelihood *a priori* would be like using a coin, with the same likelihood of getting heads or getting tails, when obtaining the class prediction.

|  | PLSR | LR | LF | SVM | Filtering (%) |
|---|---|---|---|---|---|
| No filtering | 57.19% | 59.92% | 53.64% | 56.64% | About 0% |
| Filtering | 80.86% | 80.01% | 74.55% | 76.01% | About 70% |

This table shows how the AUC value is kept between 53% and 60% when no filtering is applied during training. The application of filtering improves the AUC value by around 20% in each case. Hence, the last column indicates that by not applying filtering, the number of examples removed from the selection is 0% and by applying filtering, around 70% are removed for the thresholds $f_i = 0.75$ and $f_d = 4.0$.

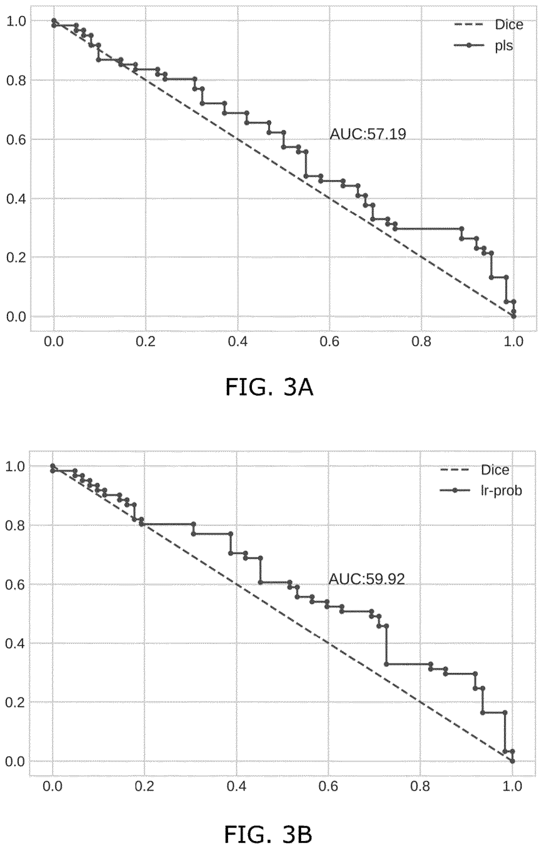

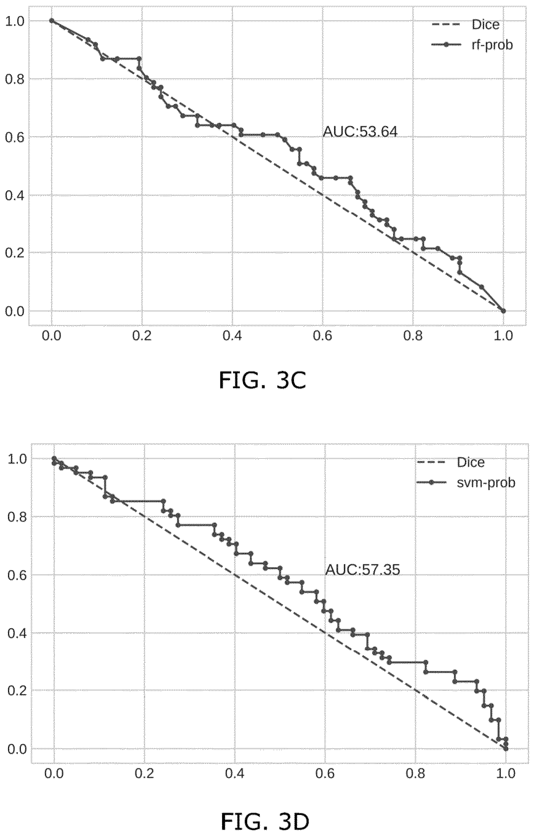

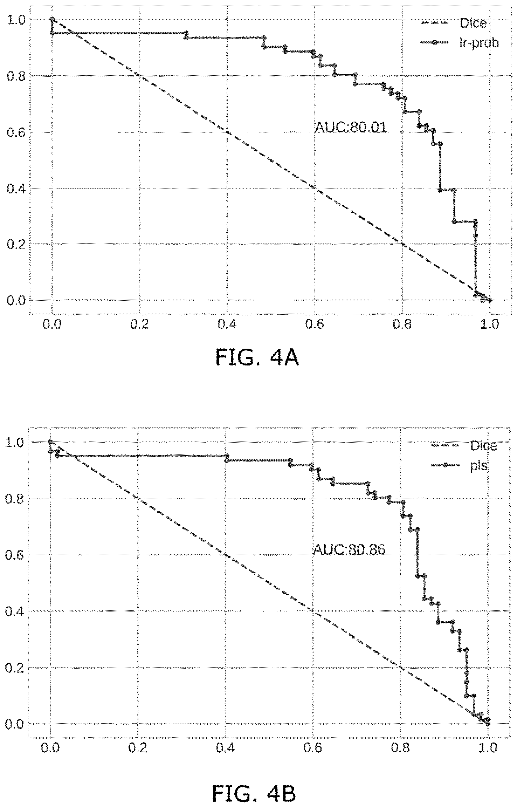

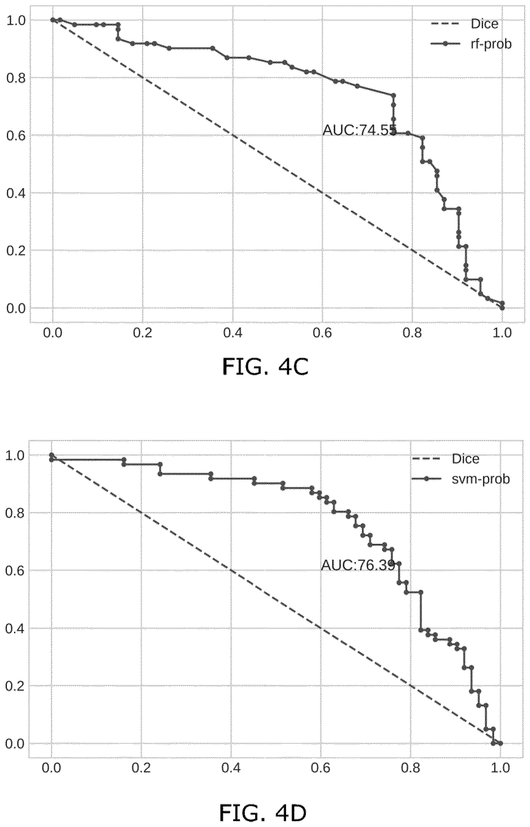

These same experiments give rise to the results shown in Figures 3A, 3B, 3C, and 3D respectively, where the figures show specificity on the x-axis and sensitivity on the y-axis during the training phase. The dashed diagonal line with the label "Dice", which goes from coordinates (0,1) to (1,0), allows observing how much the curves separate for each of the algorithms used: the PLSR algorithm with the label "pls" in Figure 3A, the LR algorithm with the label "lr-prob" in Figure 3B, the RF algorithm with the label "rf-prob" in Figure 3C, and the SVM algorithm with the label "svm-prob" in Figure 4D. In all the cases, the curves are very close to the dashed line and with a low AUC value. The label "Dice" refers to the result that is equivalent to what would be obtained when rolling dice, that is, the result would be pure chance.

In contrast, when filtering is applied, these same curves are shown in Figures 4A to 4D where the curves now separate significantly from the dashed line, raising the AUC value.

That is, with the use of a set of filtered data according to the measurement of the modified distance $d = (x - x_d)^T \beta \Omega (x - x_d)$ and with the specific threshold values which allow removing samples very close to the boundary and very far away from the group, a more sensitive classifier has been obtained. In these cases, the labels used assign a 0 for the first class and a 1 for the second class.

*Validation phase*

After a training phase, a validation phase should be carried out with a set of patients other than the set of patients used in the training phase, and where these patients are labeled after having been previously diagnosed by means of, for example, invasive techniques. In the validation phase, the set of patients is a distinct set, that is, they are patients which belong to either the first class or to the second class.

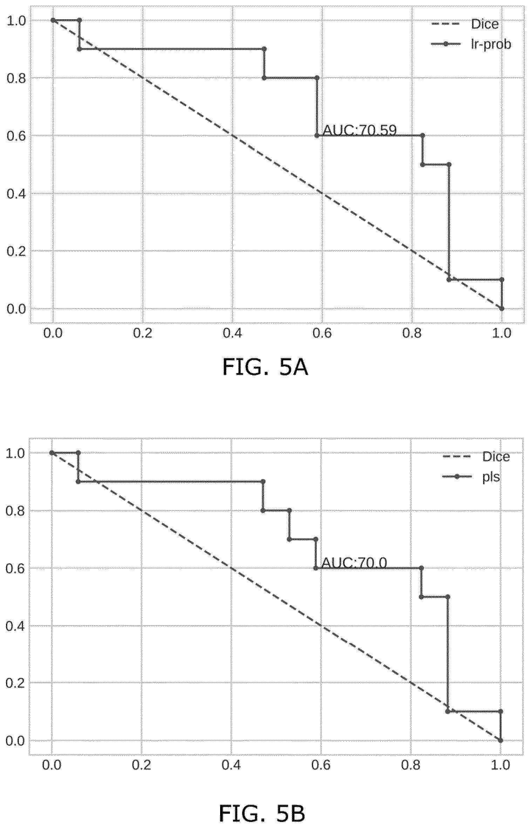

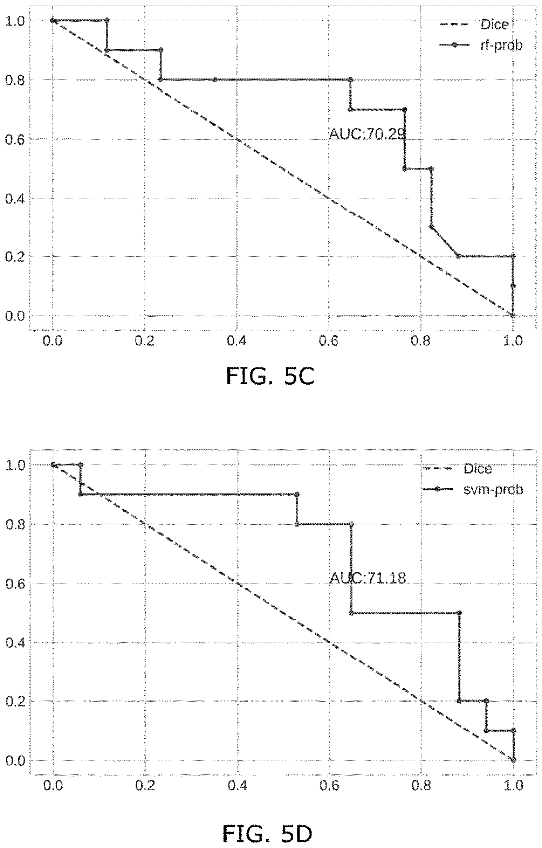

The following validation phase corresponds to Figures 5A and 5D. The four figures show sensitivity versus specificity for each projection algorithm. The value obtained for patients set aside for the validation phase gave an AUC value between 70.00 and 70.59.

On this same set of patients, an additional filtering has now been applied, where the prediction is provided for patients having conditions close to those used during training, and it is done by rejecting during the classification phase those patients having a distance to the boundary that is greater than a threshold value.

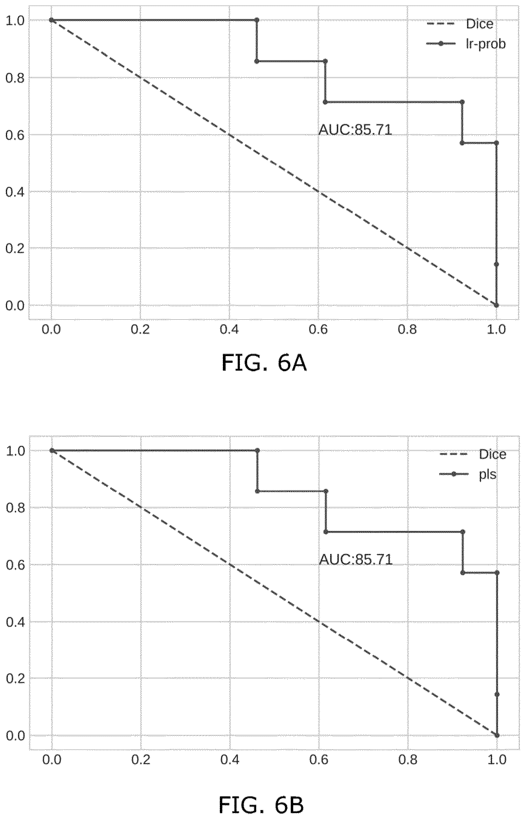

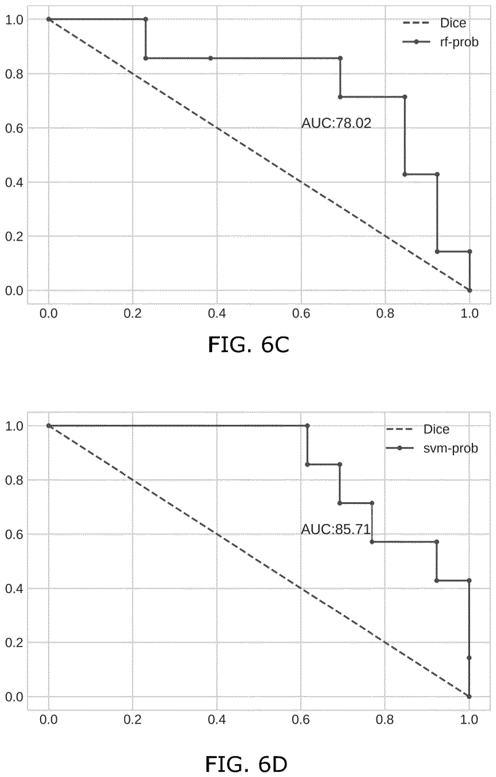

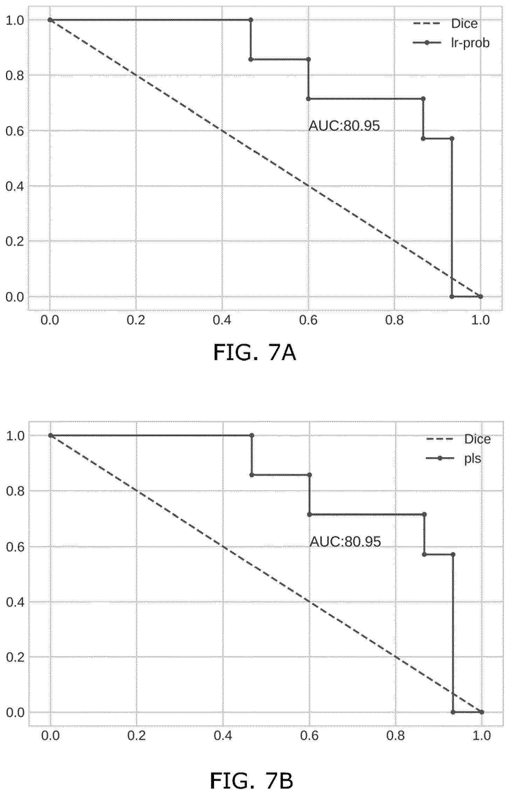

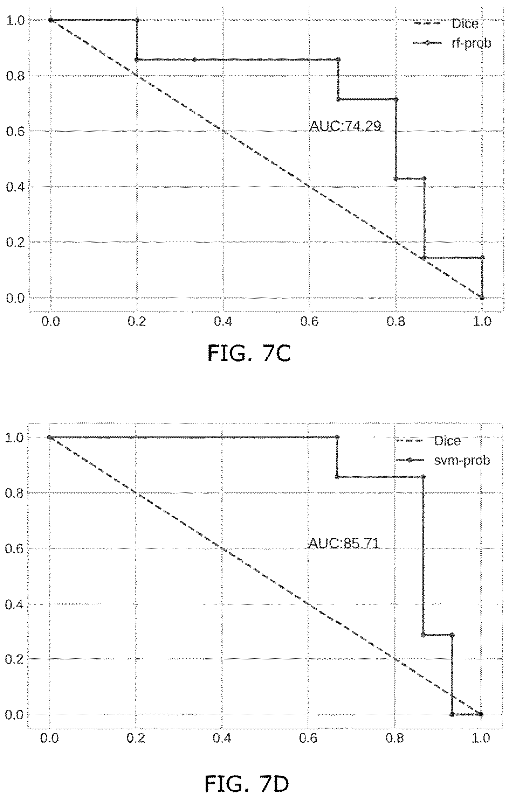

Figures 6-6D show the results for the four projection algorithms for a threshold value of distance to the boundary with $\tau_2 > 0.75$, with 0.75 being the threshold value, and Figures 7A to 7D show the results with a higher threshold value, $\tau_2 > 0.85$. That is, during the classification method, the filtering of points is based on how close they are or are not to the training sample and if they are individuals that are very far away, they are considered individuals not seen in the training and therefore not suitable for classification.

The first one accepts for being classified 20 out of 27 patients (about a 74% of classified individuals with respect to the total), and the second one accepts for being classified 22 out of 27 patients (about 81% of classified individuals with respect to the total).

The results show that the classifier described according to the invention allows a classification of patients who could belong to more than one class, that is, their belonging to one class or the other can be defined by a certain percentage of belonging. This could be confusing in the phase of determining a discriminant hyperplane.

These results have been improved considerably by adding a filtering phase during the training phase where patients are removed when the projected sample falls very close to the boundary, or patients are removed when the projected sample falls very far away, or they are both removed.

After applying this filtering on the selected patients and using a smaller number of patients, i.e., those which verify that the distance is not very close to the boundary and are not very far away, the effectiveness of the classifier has increased substantially.

It was seen in the validation phase with a set of distinct patients, i.e., patients that only belong to one class, that effectiveness using the filtering operation is very high.

**Claims**

1. A training method for training a machine learning machine for classifying urology patients into at least a first class of patients with bladder outlet obstruction (BOO) and a second class of patients with detrusor underactivity (DU), wherein the method comprises the following steps for adapting the machine learning machine:

   a) selecting a set of patients who have either been diagnosed as being in the first class or else have been diagnosed as being in the second class;
   b) receiving data representative of a uroflow measurement during urination for each selected patient, the measurement being proportional to flow over time;
   c) processing, by means of a computational system, the data received in the previous step, such that the data representative of the uroflow measurement is sampled over time, generating a vector (v) of a pre-established dimension, and wherein the set of vectors (v) of the selected set of patients results in a plurality of points in a multidimensional space, labeling each point according to the class of the patient to which it belongs;
   d) determining, in any order, by means of a computational system:

      - a discriminant hyperplane in the multidimensional space, such that it establishes a classification of the first class and the second class;

- a projection of the plurality of vectors (v) in an orthogonal projection subspace, the projection subspace in turn being orthogonal to the discriminant hyperplane, and wherein the intersection between the projection subspace and the hyperplane establishes a boundary between classes;

e) determining those samples formed by vectors (v) projected in the projection subspace located at a distance from the boundary that is less than a pre-established threshold value, and removing said samples from the set of samples;

f) training the machine learning machine such that, for each sample of the orthogonal projection subspace, the classification it provides is the class with which it is labeled, wherein the samples are the resultants in the previous step.

2. The training method according to claim 1, wherein the set of patients selected in step a) do not belong to both classes at the same time.

3. The method according to any of the preceding claims, wherein, during training of the machine learning machine, vectors (v) related to patients that correspond to samples of the projection space and verify that the distance to the boundary is greater than a pre-established value are discarded, as well as the sample.

4. A method for classifying a urology patient into at least the first class or the second class, the method using a computational system adapted to run a machine learning machine trained according to any of claims 1 to 3, the computational system storing at least the following parameters determined in the training:

- parameters defining the multidimensional space,
- the orthogonal projection subspace, and
- the discriminant hyperplane and/or the boundary separating the projection space,

wherein the method comprises carrying out, by means of the computational system, the following steps:

1. receiving data representative of a uroflow measurement during urination of a patient, the measurement being proportional to flow over time;
2. processing the data from the previous step, such that the data is sampled over time, generating a vector (v) of the dimension of the multidimensional space;
3. projecting the vector (v) of the multidimensional space to which the vector (v) in the orthogonal projection subspace belongs, giving rise to a projected sample;
4. classifying the patient by means of the machine learning machine, using as input datum the sample projected on the orthogonal projection subspace depending on the side of the boundary established by the discriminant hyperplane where said projected sample is located;
5. providing as output the class to which the datum representative of the uroflow measurement belongs.

5. A system for classifying a urology patient into at least the first class or the second class, the system comprising:

- a computational system adapted to run a machine learning machine trained according to any of claims 1 to 3 and storing at least the following parameters determined in the training:

parameters defining the multidimensional space,
the orthogonal projection subspace, and
the discriminant hyperplane and/or the boundary separating the orthogonal projection space,

- the computational system being further adapted to carry out the following steps:

i. receiving data representative of a uroflow measurement during urination of a patient, the measurement being proportional to flow over time;
ii. processing the data from the previous step, such that the data is sampled over time, generating a vector (v) of the dimension of the multidimensional space;
iii. projecting the vector (v) of the multidimensional space to which the vector (v) belongs in the orthogonal projection subspace, giving rise to a projected sample;
iv. classifying the patient by means of the machine learning machine, using as input datum the sample projected on the orthogonal projection subspace depending on the side of the boundary established by the discriminant hyperplane where said projected sample is located;

v. providing as output the class to which the datum representative of the uroflow measurement belongs.

6. The method according to claim 4 and the system according to claim 5, further comprising measuring equipment adapted to carry out a uroflow measurement during urination of the patient, the measuring equipment being adapted to generate data representative of a signal proportional to flow over time.

7. The system according to any of the preceding claims, wherein the data representative of the signal measured by the uroflow measuring equipment is normalized by scaling the signal as a function of time to a pre-established period of time (T).

8. The system according to the preceding claim, wherein the scaling is carried out by means of the computational system based on the sampled signal according to the following steps:

- carrying out interpolation of the sampled signal by providing an interpolating function in the period of time in which the measurement is non-zero, preferably by means of piecewise polynomial interpolation or spline techniques;
- scaling the interpolating function from the period of measurement to the period pre-established as normalized (T);
- resampling the interpolating function scaled in the previous step.

9. The system according to any of the preceding claims, wherein the vector (v) is dimensionally expanded by means of the computational system by adding the result of sampling one or more of the following functions generated from the measured signal interpreted as a function of time:

- the time derivative function of order n of the measured signal, with $n$ being a natural number;
- the Fourier transform of the measured signal;
- one or more wavelet transforms of the measured signal.

10. The method according to claim 4 and the system according to any of claims 5 to 9, wherein vectors (v) projected on the orthogonal projection subspace and which verify that the distance to the boundary is less than a pre-established threshold value are considered unclassifiable.

11. The method according to claim 4 and the system according to any of claims 5 to 9, wherein during the classification of a patient, vectors (v) projected on the orthogonal projection subspace and which verify that the distance to the boundary is greater than a pre-established threshold value are considered unclassifiable.

12. The method according to any of claims 1 to 4 and the system according to any of claims 5 to 11, wherein the measurement of the distance $d$ between a point $x$ in the multidimensional space and a point in the discriminant hyperplane $x_d$ is determined by means of:

$$d = (x - x_d)^T \beta \Omega (x - x_d)$$

where $\beta$ is a diagonal matrix with the dimension n of the multidimensional space and the elements $\beta_{ii}$ of the diagonal comprising weights assigned to each dimension; and $\Omega$ is a symmetrical square matrix $nxn$ where each element $\Omega_{ij}$ is the positive or negative quantitative relationship between dimensions $i$ and $j$.

13. The method and the system according to the preceding claim, wherein $\beta$ is the identity matrix $I$ and $\Omega = \Sigma^{-1}$, with $\Sigma$ being the covariance matrix.

14. The method and the system according to any of claims 12 to 13, wherein $x_d$ is estimated by $\mu$, the vector of dimension n representing the mean of all the individuals for each variable.

15. A computer program comprising instructions which, when the program is run by the computer, causes the computer to carry out steps b) to f) according to any of claims 1 to 3.

16. A computer program comprising instructions which, when the program is run by the computer, causes the computer to carry out steps 1 to 5 according to claim 4.

FIG. 1A

FIG. 1B

FIG. 1D

FIG. 1C

EP 4 687 153 A1

FIG. 2

FIG. 3A

FIG. 3B

## FIG. 3C

## FIG. 3D

FIG. 4A

FIG. 4B

FIG. 4C

FIG. 4D

FIG. 5A

FIG. 5B

FIG. 5C

FIG. 5D

FIG. 6A

FIG. 6B

FIG. 6C

FIG. 6D

FIG. 7A

FIG. 7B

FIG. 7C

FIG. 7D

# EP 4 687 153 A1

**EUROPEAN SEARCH REPORT**

Europäisches Patentamt
European Patent Office
Office européen des brevets

Application Number

EP 24 38 2866

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YOSHIHISA MATSUKAWA ET AL: "Characteristics of uroflowmetry patterns in men with detrusor underactivity revealed by artificial intelligence", INTERNATIONAL JOURNAL OF UROLOGY, CHURCHILL LIVINGSTONE, TOKYO, JP, [Online] vol. 30, no. 10, 21 June 2023 (2023-06-21) , pages 907-912, XP072512059, ISSN: 0919-8172, DOI: 10.1111/IJU.15233 [retrieved on 2025-01-15] | 1-16 | INV. G16H50/20 G16H50/70 ADD. A61B5/20 A61B5/00 |
| Y | * the whole document * * in particular * * Methods, Results and Discussion sections; tables 1, 2 * ----- | 1-16 | |
| X | US 2022/215960 A1 (LEE KYU SUNG [KR] ET AL) 7 July 2022 (2022-07-07) * the whole document * * in particular * * paragraph [0047]; figures 4,5,9 * ----- -/-- | 1-16 | |
| | | | TECHNICAL FIELDS SEARCHED (IPC) G16H A61B |

The present search report has been drawn up for all claims

| Place of search | Date of completion of the search | Examiner |
|---|---|---|
| Munich | 16 January 2025 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt

European Patent Office

Office européen des brevets

# EUROPEAN SEARCH REPORT

**Application Number**

EP 24 38 2866

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KNORR JACOB M. ET AL: "Machine Learning and Artificial Intelligence to Improve Interpretation of Urodynamics", CURRENT BLADDER DYSFUNCTION REPORTS, [Online] vol. 19, no. 1, 13 January 2024 (2024-01-13), pages 44-53, XP093239780, Boston ISSN: 1931-7212, DOI: 10.1007/s11884-023-00734-2 Retrieved from the Internet: URL:https://link.springer.com/content/pdf/10.1007/s11884-023-00734-2.pdf> [retrieved on 2025-01-15] * the whole document * * in particular * | 1-16 | |
| Y | TSAI CHUNG-YOU ET AL: "Building Dual AI Models and Nomograms Using Noninvasive Parameters for Aiding Male Bladder Outlet Obstruction Diagnosis and Minimizing the Need for Invasive Video-Urodynamic Studies: Development and Validation Study", JOURNAL OF MEDICAL INTERNET RESEARCH, [Online] vol. 26, 20 March 2024 (2024-03-20), page e58599, XP093239809, CA ISSN: 1438-8871, DOI: 10.2196/58599 Retrieved from the Internet: URL:https://www.jmir.org/2024/1/e58599/PDF > [retrieved on 2025-01-15] * the whole document * * in particular * * results section; figure 1; tables 1-3 * | 1-16 | **TECHNICAL FIELDS SEARCHED (IPC)** |

The present search report has been drawn up for all claims

| Place of search | Date of completion of the search | Examiner |
|---|---|---|
| Munich | 16 January 2025 | Rákossy, Z |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 38 2866

This annex lists the patent family members relating to the patent documents cited in the above-mentioned European search report.
The members are as contained in the European Patent Office EDP file on
The European Patent Office is in no way liable for these particulars which are merely given for the purpose of information.

16-01-2025

| Patent document cited in search report | Publication date | Patent family member(s) | | Publication date |
|---|---|---|---|---|
| US 2022215960 A1 | 07-07-2022 | JP | 7278415 B2 | 19-05-2023 |
| | | JP | 2022529460 A | 22-06-2022 |
| | | KR | 20200122084 A | 27-10-2020 |
| | | US | 2022215960 A1 | 07-07-2022 |
| | | WO | 2020213826 A1 | 22-10-2020 |

EPO FORM P0459

For more details about this annex : see Official Journal of the European Patent Office, No. 12/82